# EUROPEAN PATENT SPECIFICATION

(11) **EP 1 247 489 B1**
(45) Date of publication and mention of the grant of the patent: **13.06.2007**
(21) Application number: 01303196.8
(22) Date of filing: 04.04.2001
(51) Int. Cl.: A61B 5/15

(54) **Blood sampling device**
Blutentnahmevorrichtung
Dispositif de prelevement sanguin

(43) Date of publication of application: 09.10.2002
(73) Proprietor: HTL - STREFA Spolka z o.o., 95-035 Ozorkow (PL)
(72) Inventor: Wyszogrodzki, Wojciech, Warzawa (PL)
(74) Representative: Fenlon, Christine Lesley

(56) References cited:
- EP-A- 1 142 534
- US-A- 5 356 420
- US-A- 5 540 709
- US-A- 6 053 930
- US-B1- 6 248 120

## Description

The subject of the present invention is a puncturing device in particular for puncturing skin of a patient in order to take a blood sample for diagnostic purposes.

U.S.Patent specification No. 4,139,011 discloses a device for puncturing skin of a patient, the device comprising a housing in the form of a sleeve closed at one end and narrowing at the other end, the sleeve having a small opening. Inside the housing there is a slidably mounted rod which, at one end, terminates in a slider positioned inside the housing. At the other end there is a rod comprising an elastic arm terminating in a holding tooth, which rests on the edge of the opening in the housing. Between the rod and the bottom of the sleeve a spring is positioned and, at the other end of the slider, a puncturing insert is provided.

A device for puncturing comprising a sleeve and a push element for actuating the device positioned at one end of the sleeve is known from the US Patent specification No. 5,356,420. The opposite end of the sleeve is open. Inside the sleeve there is a slidably mounted piston, which at the end closer to the push element terminates in a head member, and which at the end closer to the open end of the sleeve terminates in a puncturing tip. Inside the sleeve, between the end face of the push element and the piston, a driving spring is located, and between the piston and the open end of the sleeve there a return spring is positioned. The piston has, at the outer circumference thereof, wings which rest on an internal projection of the sleeve.

According to one aspect of the present invention, there is provided a sampling device comprising a sleeve, a push element mounted at one end of the sleeve, the sleeve having an opening at the opposite end thereof, a piston with a puncturing tip slideably mounted in the sleeve, a drive spring positioned between the end face of the push element and the piston and a return spring positioned inside the sleeve between the opening and the piston, characterised in that the sleeve has breakable wings directed inwardly and the piston has an external projection which rests on the wings.

Preferably, the wings are provided on a washer having an outer ring, the washer being positioned at the first end of the sleeve and the piston has an external projection which rests on the wings.

According to a further aspect of the present invention there is provided a sampling device comprising a sleeve, a push element mounted at a first end of the sleeve, the sleeve having an opening at the opposite end thereof, a piston with a puncturing tip slideably mounted in the sleeve, a drive spring positioned between the end face of the push element and the piston and a return spring positioned inside the sleeve between the opening and the piston, characterised in that piston has a circumferential flange on its outer surface that rests on the first end of the sleeve, the flange being detachable from the piston when pressure is applied to the push element.

The sampling device of the invention is a puncturing device, primarily, but not only, intended for puncturing the skin of the patient and is generally referred to hereinafter as a puncturing device.

Preferably the flange comprises a ring which is detachably joined to the piston by breakable fasteners.

The advantage of the device according to the invention is that it provides a way of rapidly and substantially painlessly piercing a patient's skin, especially the skin on a finger, ear or heel of a patient, in order, for example, to take a blood sample for diagnostic purposes. The device, due to its structure, is expendable, because after the wings are broken off, it cannot be re-used.

For a better understanding of the invention, and to show how the same may be carried into effect, reference will now be made, by way of example, to the accompanying drawings, in which:-
Fig. 1 shows a longitudinal section through a first embodiment of the device before use;
Fig. 2 shows a longitudinal section through the first embodiment after use;
Fig. 3 shows a longitudinal section through a second embodiment before use;
Fig. 4 shows a top view of the washer of the second embodiment of the device;
Fig. 5 shows a longitudinal section through a third embodiment before use;
Fig. 6 shows a top view of the piston with the flange of the third embodiment of the device;
Fig. 7 shows a longitudinal section through the third embodiment of the device after use.

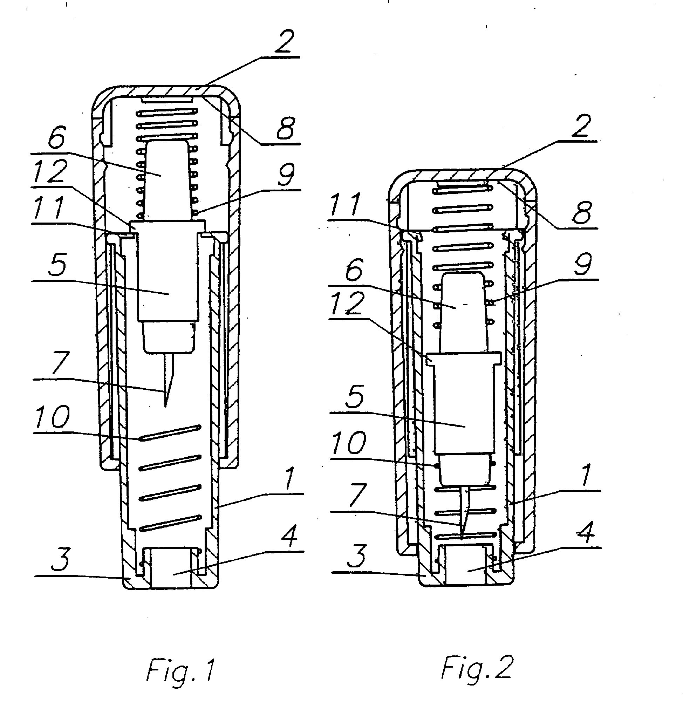

The puncturing device according to the first embodiment as shown in Figs. 1 and 2 comprises a sleeve 1 and a push element 2 mounted at one end of the sleeve 1 and surrounding substantially the entire length of the sleeve. The other end of the sleeve 1 has an opening 4. A slidably mounted piston 5 is located in the sleeve 1. At the end closer to the push element 2, the piston has a pusher 6, and at the end closer to the open end of the sleeve 1, a puncturing tip 7 is located on the piston 5. A drive spring 9 is positioned inside the sleeve 1 between the end face 8 of the push element 2 and the piston 5. A return spring 10 is positioned inside the sleeve 1 between the piston 5 and the bottom 3 of the sleeve. The upper section of the sleeve 1 has inwardly directed wings 11. A projection 12 is situated on the external surface of the piston 5.

A description of the operation of the device according to the invention follows. The arrangement of the elements of the device before use is presented in Fig. 1. As a result of the pressure applied by the drive spring 9, the projection 12 of the piston 5 rests on the inward wings 11, which are positioned at the upper part of the sleeve 1. Thus, the piston 5 with the puncturing tip 7 is held in a first stable position. Pressing the push element 2 causes squeezing of the drive spring 9 to the point where the end face of the push element 2 rests on the pusher 6 of the piston 5. Applying further pressure to the push element 2 causes the wings 11 of the sleeve 1 to be broken off by the projection 12 of the piston 5. The drive spring 9 actuates the piston 5 causing the puncturing tip 7 to extend through the opening 4 at the bottom of the sleeve 1 and puncture the skin. Subsequently, the return spring 10 withdraws the piston 5 with the puncturing tip 7 assuming a second stable position inside the sleeve 1. The position of the elements after use is illustrated in Fig. 2. Re-use of the device once the wings 11 have been broken off is impossible.

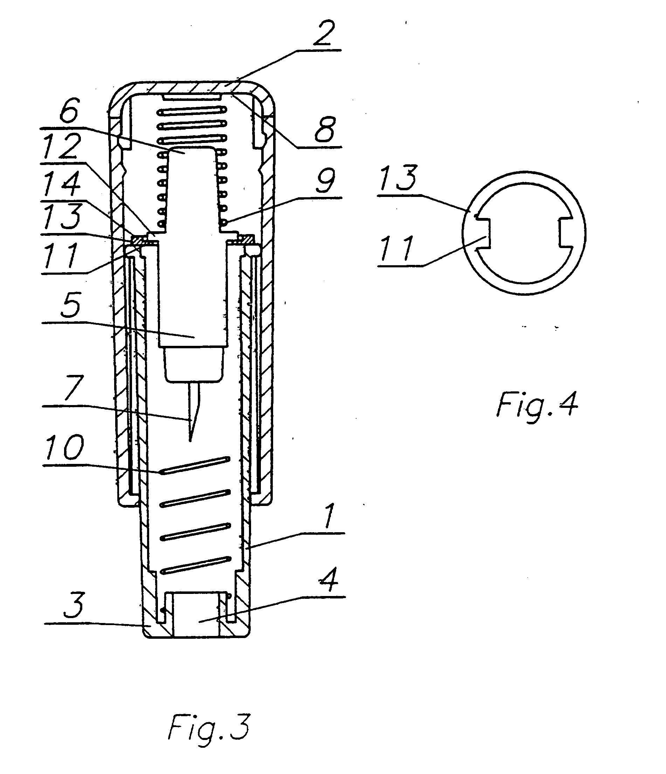

A second embodiment of the puncturing device according to the invention is shown in Figs. 3 and 4. Reference numerals in respect of the main elements of the device are identical to those used in Figs. 1 and 2. This embodiment is characterised by the fact that the wings 11 together with an outer ring 13 form a washer 14 mounted on the upper part of the sleeve 1 the projection 12 of the piston 5 resting on the wings 11 of the washer 14. Operation of the device is identical as presented in relation to Figs. 1 and 2.

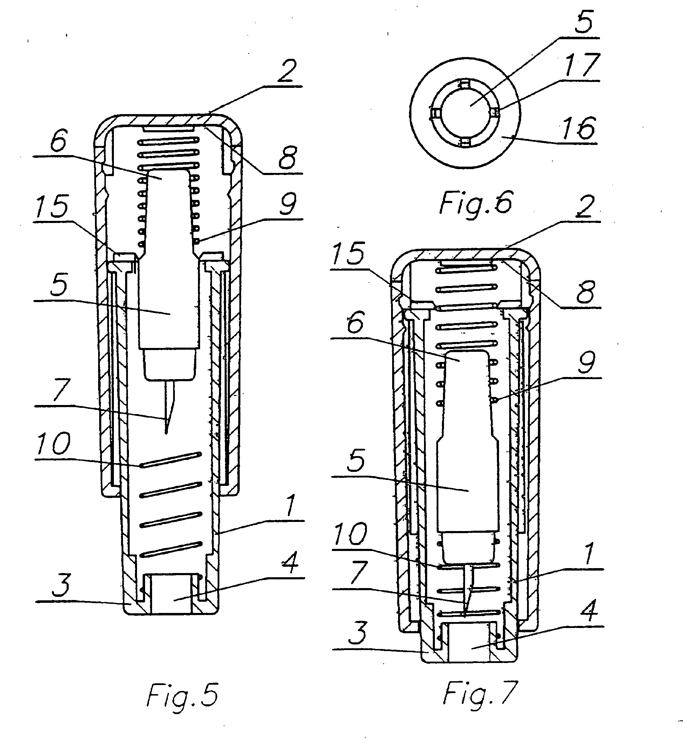

A third embodiment of the device according to the invention is illustrated by Figs 5 to 7. Reference numerals are identical to those in Figs. 1 to 4. In this embodiment the piston 5 comprises a flange 15 on its outer rim resting on the upper section of the sleeve 1. Flange 15 includes a ring 16 and fasteners 17. The fasteners 17 detachably connect the ring 16 to the piston 5. The device of this embodiment operates in a similar way to that described in relation to Figs. 1 and 2 but with the difference that when the pressure is applied to the push element 2 and further to the piston 5, the fasteners 17 are broken off causing the piston 5 to detach from the flange 15.

## Claims

1. A sampling device comprising a sleeve (1), a push element (2) mounted at one end of the sleeve, the sleeve (1) having an opening (4) at the opposite end thereof, a piston (5) with a puncturing tip (7) slidably mounted in the sleeve (1), a drive spring (9) positioned between the end face (8) of the push element (2) and the piston (5) and a return spring (10) positioned inside the sleeve between the opening (4) and the piston (5), **characterised in that** the sleeve (1) has breakable wings (11) directed inwardly and the piston (5) has an external projection (12) which rests on the wings (11).

2. A sampling device as claimed in claim 1, wherein the breakable wings (11) are provided on a washer (14) having an outer ring, the washer (14) being positioned at the first end of the sleeve (1) and the piston (5) has an external projection (12) which rests on the wings (11).

3. A sampling device comprising a sleeve (1), a push element (2) mounted at a first end of the sleeve (1), the sleeve (1) having an opening (4) at the opposite end thereof, a piston (5) with a puncturing tip (7) slideably mounted in the sleeve (1), a drive spring (9) positioned between the end face (8) of the push element (2) and the piston (5) and a return spring (10) positioned inside the sleeve (1) between the opening (4) and the piston (5), **characterised in that** piston (5) has a circumferential flange (15) on its outer surface that rests on the first end of the sleeve (1), the flange (15) being detachable from the piston (5) when pressure is applied to the push element (2).

4. Device according to claim 3 wherein the flange (15) comprises a ring (16) which is detachably joined to the piston (5) by breakable fasteners (17).

## Patentansprüche

1. Sammelvorrichtung, umfassend eine Hülse (1); ein Schiebeelement (2), montiert an einem Ende der Hülse, wobei die Hülse (1) am gegenüberliegenden Ende eine Öffnung (4) besitzt; einen Kolben (5) mit einer Anstichspitze (7), verschieblich montiert in der Hülse (1); eine Antriebsfeder (9), die angeordnet ist zwischen der Stirnfläche (8) des Schiebeelements (2) und dem Kolben (5); sowie eine Rückschubfeder (10), angeordnet in der Hülse zwischen der Öffnung (4) und dem Kolben (5), **dadurch gekennzeichnet, dass** die Hülse (1) abbrechbare Flügel (11) besitzt, welche nach innen gerichtet sind und der Kolben (5) einen außenliegenden Vorsprung (12) besitzt, der auf den Flügeln (11) sitzt.

2. Sammelvorrichtung nach Anspruch 1, wobei die abbrechbaren Flügel (11) auf einer Beilagscheibe (14) vorgesehen sind, umfassend einen äußeren Ring, wobei die Beilagscheibe (14) am ersten Ende der Hülse (1) angeordnet ist und der Kolben (5) einen außenliegenden Vorsprung (12) aufweist, der auf den Flügeln (11) sitzt.

3. Sammelvorrichtung, umfassend eine Hülse (1), ein Schiebeelement (2), montiert am ersten Ende der Hülse (1), wobei die Hülse (1) eine Öffnung (4) am gegenüber liegenden Ende besitzt, einen Kolben (5) mit einer Anstichspitze (7), verschieblich montiert in der Hülse (1), eine Antriebsfeder (9), angeordnet zwischen der Stirnfläche (8) des Schiebeelements (2) und dem Kolben (5) sowie eine Rückfeder (10), angeordnet innen in der Hülse (1) zwischen der Öffnung (4) und dem Kolben (5), **dadurch gekennzeichnet, dass** der Kolben (5) einen kreisumfänglichen Flansch (15) auf seiner Außenfläche besitzt, der aufsitzt auf dem ersten Ende der Hülse (1), wobei der Flansch (15) lösbar ist vom Kolben (5), wird auf das Schiebeelement (12) ein Druck aufgebracht.

4. Sammelvorrichtung nach Anspruch 3, wobei der Flansch (15) einen Ring (16) umfasst, der mit dem Kolben (5) über abbrechbare Befestigungsmittel (17) lösbar verbunden ist.

## Revendications

1. Dispositif de prélèvement comprenant un manchon (1), un élément pousseur (2) monté à une extrémité du manchon, le manchon (1) comportant une ouverture (4) à son extrémité opposée, un piston (5) muni d'un embout de perforation (7) monté de façon glissante dans le manchon (1), un ressort d'entraînement (9) positionné entre la face d'extrémité (8) de l'élément pousseur (2) et le piston (5) et un ressort de rappel (10) positionné à l'intérieur du manchon entre l'ouverture (4) et le piston (5), **caractérisé en ce que** le manchon (1) comporte des volets cassables (11) orientés vers l'intérieur et le piston (5) a une protubérance externe (12) qui repose sur les volets (11).

2. Dispositif de prélèvement selon la revendication 1, dans lequel les volets cassables (11) sont prévus sur une rondelle (14) comportant une bague extérieure, la rondelle (14) étant positionnée à la première extrémité du manchon (1) et le piston (5) a une protubérance externe (12) qui repose sur les volets (11).

3. Dispositif de prélèvement comprenant un manchon (1), un élément pousseur (2) monté à une première extrémité du manchon (1), le manchon (1) comportant une ouverture (4) à son extrémité opposée, un piston (5) muni d'un embout de perforation (7) monté de façon glissante dans le manchon (1), un ressort d'entraînement (9) positionné entre la face d'extrémité (8) de l'élément pousseur (2) et le piston (5) et un ressort de rappel (10) positionné à l'intérieur du manchon (1) entre l'ouverture (4) et le piston (5), **caractérisé en ce que** le piston (5) comporte une bride périphérique (15) sur sa surface extérieure qui repose sur la première extrémité du manchon (1), la bride (15) étant détachable du piston (5) lorsqu'une pression est appliquée à l'élément pousseur (2).

4. Dispositif de prélèvement selon la revendication 3, dans lequel la bride (15) comprend un anneau (16) qui est lié de façon détachable au piston (5) par des fixations cassables (17).
